# EUROPÄISCHE PATENTSCHRIFT

(11) **EP 0 895 797 B1**
(45) Veröffentlichungstag und Bekanntmachung des Hinweises auf die Patenterteilung: **12.05.2004**
(21) Anmeldenummer: 98114877.8
(22) Anmeldetag: 07.08.1998
(51) Int. Cl.: A63H 3/00, A63H 3/16

(54) **Spielfigur**
Toy figure
Figure jouet

(30) Priorität: 07.08.1997 DE 19734150
(43) Veröffentlichungstag der Anmeldung: 10.02.1999
(73) Patentinhaber: Lippold, Michael, 21335 Lüneburg (DE)
(72) Erfinder: Lippold, Michael, 21335 Lüneburg (DE)
(74) Vertreter: Vonnemann, Gerhard, Dr.-Ing.

(56) Entgegenhaltungen:
- DE-A- 2 458 084
- US-A- 4 820 234
- US-A- 5 094 621
- US-A- 5 203 708

## Beschreibung

Die Erfindung betrifft ein System mit mindestens einer Spielfigur als Mittel zur Symbolisierung und Visualisierung des innerseelischen Geschehens in Einzelpersonen zur Übung der Selbstwahrnehmung und Bewußtmachung von Konflikten in Zweierbeziehungen und Gruppenbeziehungen, gemäß dem Oberbegriff des Patentanspruchs 1.

Eine Spielfigur vergleichbarer Art ist beispielsweise aus der US-A-5 094 621 zu entnehmen.

Es ist bekannt, daß es verschiedene psychologische Literaturen zur Einübung und Förderung der Selbstwahrnehmung gibt. Diese Informationen sind aber nur mit einem erheblichen Aufwand an Zeit und großer kognitiver Verarbeitung und Auseinandersetzung zu erschließen. Der Zugang zu einem förderlichen und differenzierten Prozeß der Selbstwahrnehmung erscheint auf diesem Hintergrund nur mit großer Motivation möglich.

Aus der US 5,094,621 ist eine therapeutische Puppe mit menschenähnlichem Äußeren bekannt, die einen durch eine Tür zugänglichen Hohlraum in ihrem Brustbereich aufweist, in den Symbolträger platziert werden können. Diese bestimmte Süchte symbolisierenden Symbolträger sollen erfindungsgemäß anschließend durch einen nichteinsehbaren Kanal aus dem Brustbereich der Puppe verschwinden und so dem Benutzer dabei helfen, seine Sucht zu überwinden. Nachteilig an dieser Puppe ist, dass diese aufgrund ihrer Ausgestaltung lediglich zur Suchtbekämpfung ausgelegt ist und dem Benutzer das Bewusstwerden seiner inneren Vorgänge nicht ermöglicht.

Aus der US 5,203,708 ist eine einen Hohlraum aufweisende menschenähnliche Figur bekannt, die eine weibliche mythische Schöpferin darstellen und zum Spielen, zum Lernen und zu therapeutischen Zwecken dienen soll. Dabei soll sie vor allem im therapeutischen Rollenspiel als Projektionsfigur für Gedanken und Gefühle dienen. Sie bietet einem Benutzer jedoch keinerlei Möglichkeit, sich selber rational über dessen innere Vorgänge bewusst zu werden oder selbständig die Selbstwahrnehmung zu üben.

Aus der US 4,820,234 ist eine zusammensteckbare Figur mit austauschbaren Gesichtselementen bekannt, bei der durch Drehung der Gesichtselemente in lustiger Weise die momentane Stimmung des Benutzers oder einer von diesem beobachteten Person wiedergegeben werden soll. Nachteilig an dieser Figur ist, dass sie dem Benutzer keine Hilfsmittel an die Hand gibt, sich über seine inneren Vorgänge bewusst zu werden, sie dient lediglich Unterhaltungszwecken.

Aus der DE P24 58 084.5 ist schließlich eine Spielfigur aus mehreren, über Verbindungsmittel miteinander verbindbaren Teilstücken bekannt, die jedoch lediglich für Kinderspielzwecke geeignet ist.

Der Erfindung liegt also das Problem zugrunde, daß keine hinreichenden Mittel zur Verfügung stehen, die es Menschen möglich macht, auf einfache Art und Weise ihr innerseelisches Geschehen symbolisch zu visualisieren und somit einen förderlichen effektiven Selbstklärungsprozeß zu fördern.

Dieses Problem wird durch die Merkmale des Patentanspruchs 1 gelöst. Diese Spielfigur, die auf einfacher und gut verständlicher Grundlage die Selbstwahrnehmung fördert, müßte für viele Menschen zugänglich gemacht werden. Mit Hilfe dieser Spielfigur, die das innere Geschehen visualisiert und in spielerischer Anleitung in einfacher und effektiver Form deutlich macht, wird der wichtige Prozeß der Selbstwahrnehmung und Selbstklärung unterstützt. Diese Spielfigur bietet also die Möglichkeit Selbstexploration interessierten Menschen dies mit angemessenem Aufwand tun zu können. Der selbstklärerische Prozeß wird hierbei durch die Visualisierung der eigenen Wünsche/Bedürfnisse und Stimmen zum gestellten Problem erkannt.
Für den Bereich der Psychologie wird das Prinzip der Visualisierung leider viel zu wenig für den Selbstwahrnehmungsprozeß und Selbstklärungsprozeß des Menschen eingesetzt. Diese beschriebene Visualisierungsfigur gibt nun die Möglichkeit, jedem interessierten Benutzer oder einer Gruppe von Benutzern, die einen klärungsbedürftigen innerseelischen Zustand empfinden, zum Beispiel in einem Entscheidungskonflikt, zu unterstützen. Der Benutzer lernt eine neue Sichtweise seines Seelenlebens kennen. Mit Unterstützung des Handbuches kann der Benutzer schnell die Benutzung dieser Spielfigur erlernen.

Vorteilhafte Ausgestaltungen sind in den Ansprüchen 2 bis 6 beschrieben.

Mit Hilfe der Spielfigur als Symbol für den Benutzer selbst, mit seinen Gedanken, Motivationen, Gefühle, können diese symbolisch, beispielsweise mit den Stimmenfiguren (21)als Symbolträgern, visualisiert werden. Der Prozeß der Selbstwahrnehmung wird spielerisch eingeübt und auf Grundlage der verschiedenen Funktionsmerkmale der Visualisierungsfiguren wirkungsvoll unterstützt.

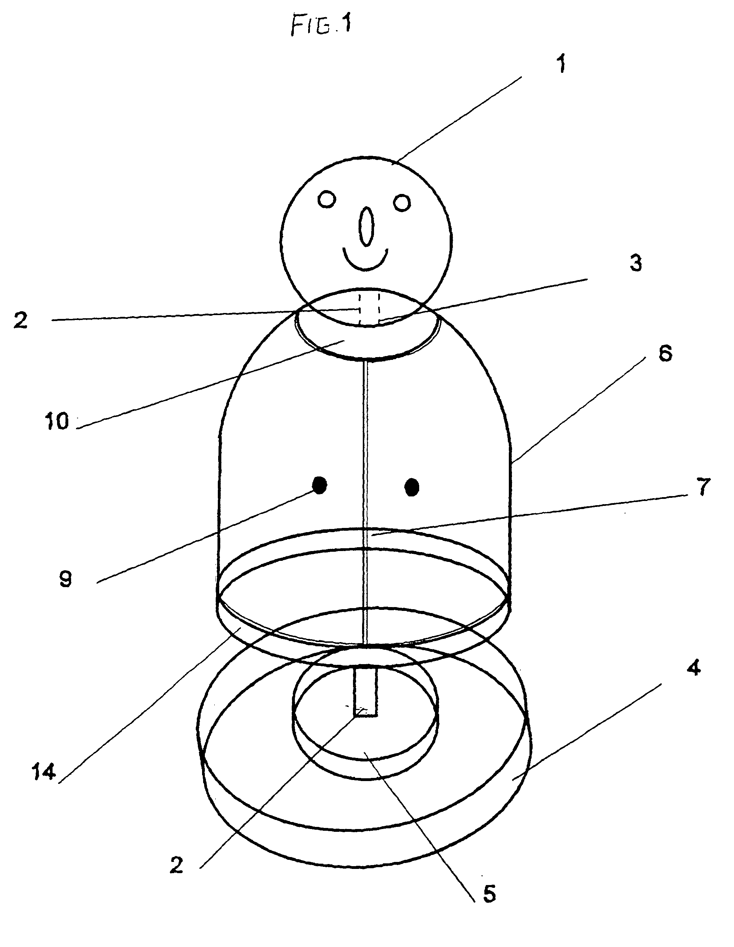

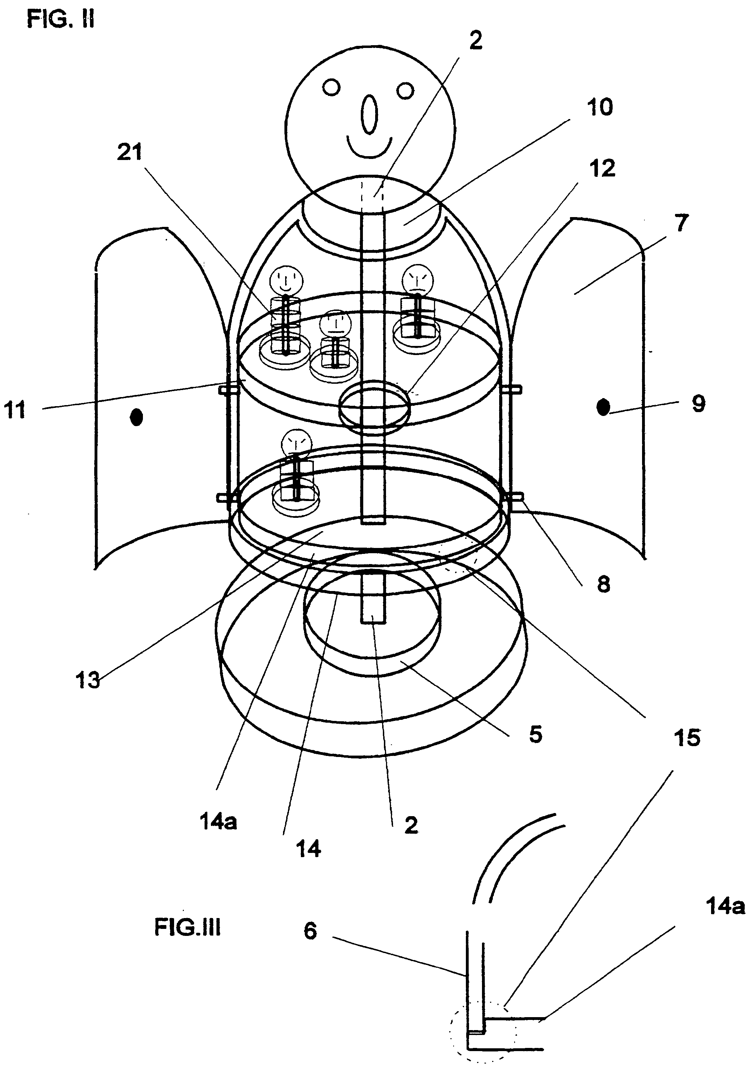

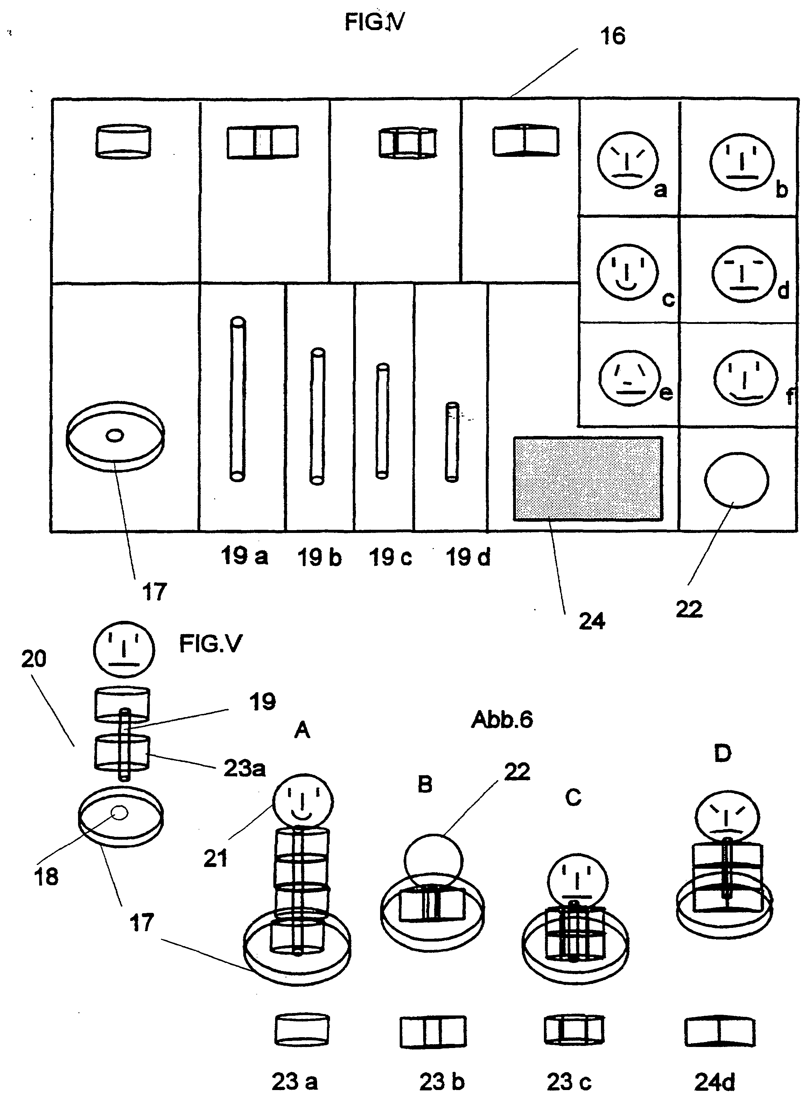

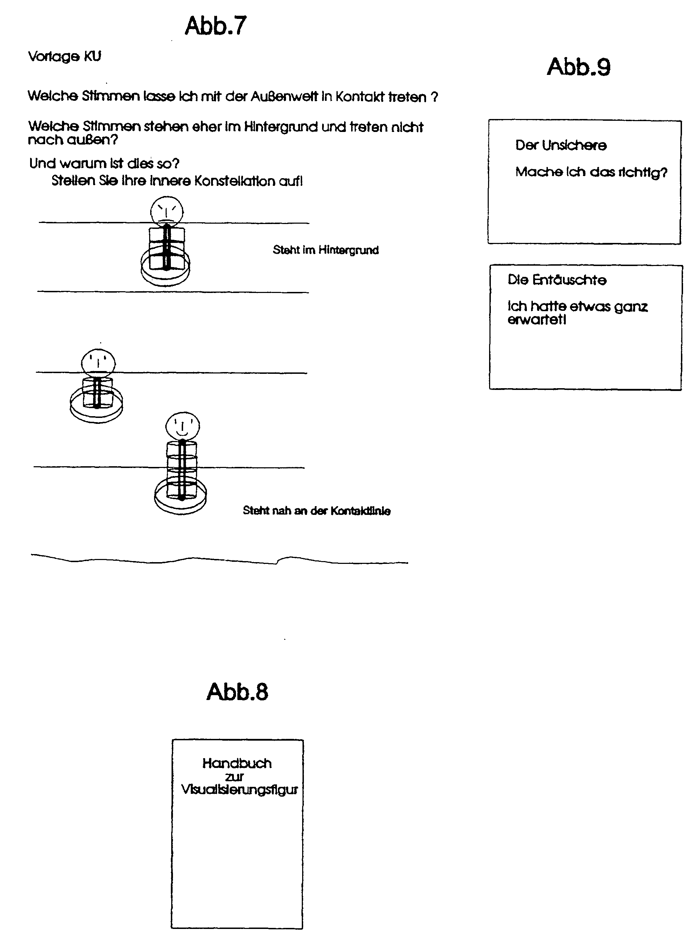

Der Benutzer hat die Möglichkeit seine verschiedenen Stimmen in seinem inneren Geschehen zu einer Konfliktsituation und zur Reflektion seiner Stimmungslage symbolisch zu visualisieren. Die Spielfigur (Fig. 1, 2) mit ihrem Hohlraum symbolisiert dabei sein Inneres. Mit Hilfe der Anweisungen und den Elementen des Zubehörkastens (16) kann der Benutzer Schritt für Schritt sein inneres Geschehen, seine inneren Konstellationen in Form von Gedanken, Gefühlen und Motivationen in Form der zusammengesteckten Stimmenfiguren (21) visualisieren. Im Innenraum der großen Visualisierungsfigur können die im lauten Selbstgespräch explorierten Stimmen symbolisch mit den zusammengesteckten Stimmenfiguren abgestellt werden (Fig. II). Mit Hilfe der verschiedenen Elemente des Zubehörkastens (16) steckt der Benutzer die einzelnen Stimmen und Personen, die in seinem Inneren vorhanden sind, zusammen. Diese können für verschiedene Aspekte von Gedanken, Gefühlen und Motivationen stehen. Hierbei kann der Benutzer mit Hilfe der Intensitätsbausteine (23 a, b, c, d) die Intensität seiner Stimmen festlegen. Er kann die Stärken seiner inneren Stimmen bestimmen durch die Anzahl der aufgesteckten Bausteine. Weiterhin bestimmt der Benutzer durch die Wahl der Form der oben genannten Intensitätsbausteine seine Gefühlsqualität seiner inneren Stimmen. Er kann hier beispielsweise zwischen vier geometrischen Formen wählen (23 a, b, c, d). Je nach Qualität der einzelnen Stimmen, Personen und Bedürfnisse wird eine entsprechende Form gewählt und somit sichtbar gemacht.

Hat der Benutzer im lauten Selbstgespräch die Intensität und Qualität seiner einzelnen Stimmen und Personen in seinem inneren Geschehen beschrieben, erkannt und visualisiert, wird dieser Figur noch ein Kugelkopf (22) aufgesetzt. Der Benutzer macht hiermit die Grundstimmung einer seiner inneren beteiligten Personen sichtbar. Er wählt aus dem Zubehörkasten (16) einen entsprechenden Gesichtsausdruck, der für die gesamte Person passen würde.

Jede Figur der inneren Konstellation wird unter dem lauten Selbstgespräch des Benutzers zusammengesteckt. Der Benutzer wählt einen Sockel (17) aus dem Zubehörkasten, wählt die Form der Intensitätssteine (23) und deren Anzahl. Hierbei wählt er den entsprechenden Haltungsstift (19), der je nach Anzahl der Intensitätsbausteine eine bestimmte Größe besitzt. Auf diesen Haltungsstift, der im Sockel (17) verankert ist, wird auch der Kugelkopf (22) mit dem entsprechenden Gesichtsausdruck aufgesetzt.
Wenn der Benutzer die innere Stimme vollständig beschrieben und zusammengesteckt hat, öffnet er die beiden Türen (7) der großen Visualisierungsfigur. Er stellt diese zusammengesteckte Figur (21) auf die mittlere Ebene (11) des Innenraums. Der Benutzer beschreibt auf einem Papierschild (24/Abb. 9) durch eine einfache Formulierung die abgestellte Figur im Inneren. Wenn nun alle Figuren zu seinem Klärungszusammenhang sichtbar geworden sind und in der großen Spielfigur abgestellt worden sind, ist die erste Phase der Selbstwahrnehmung abgeschlossen.

Er betrachtet nun seine innere Konstellation mit den verschiedenen Stimmen und Bedürfnissen. Es befinden sich nun verschiedene zusammengesteckte Figuren im Inneren der Visualisierungsfigur. Der Benutzer nimmt den zylindrischen Hohlkörper (6) von der Visualisierungsfigur und betrachtet die zusammengesteckten Figuren, die er auf der mittleren Ebene (11) abgestellt hat.
Nun kann er überlegen, welche Stimmen eher den Außenkontakt gestalten und welche Anteile eher nicht gehört, nicht wahrgenommen, nicht gelebt werden. Diese Anteile stellt er auf die untere Ebene der Spielfigur. Im weiteren Verlauf der Selbstexploration kann der Benutzer überlegen, welche Stimmen in seinem Inneren verändert und modifiziert werden können, um zu einer förderlichen Gestaltung seines inneren Erlebens und äußeren Verhaltens zu kommen.
Hierbei kann der Benutzer die Vorlage KU (Abb. 7) benutzen. Unter der Fragestellung, welche Stimmen den Kontakt mit der Außenwelt gestalten, bestimmt er, welche Stimmen ganz vorne den Außenkontakt gestalten und welche Stimmen eher im mittleren und hinteren Bereich den Kontakt gestalten, d. h. ganz selten oder gar nicht.
Der Benutzer stellt seine Figuren im lauten Selbstgespräch diese Figuren auf die Vorlage. Er kann nach weiterer Beschreibung der inneren Stimmen darüber nachdenken, welche Stimmen seines inneren Geschehens verändert und entwickelt werden können.

Die Spielfigur kann aufgrund ihrer Konstruktionsmerkmale so gestaltet werden, daß sie praktisch, funktionell handlich und dauerhaft haltbar zusammengesteckt und auseinandergenommen werden kann, so daß sie mit den anderen Elementen in einem passenden Zubehörkasten verstaut werden können, wobei der Holzkopf (1), der Hohlkörper (6), die Holzstange (2), die mittlere Ebene (11), die untere Ebene (14) und der Holzfuß (4) mit dem Verstärkungssockel (5) als Einzelteile konzipiert sind.

Die Spielfigur kann so konzipiert werden, daß zu der unteren Ebene (14 a) der mittleren Ebene (11) eine weitere Ebene zur Aufstellung von kleinen Spielfiguren (21) in der Spielfigur möglich ist, so daß insgesamt 3 Räume entstehen. Wieviele Ebenen bei der zur Visualisierung des innerseelischen Geschehens installiert werden, kann je nach Fragestellung und Fähigkeit der Benutzer variiert werden, da die Ebenen flexibel in die Figur eingelegt werden können.

Die Ebenen symbolisieren zum Beispiel die jeweilige Bewußtheit der Wünsche (Bedürfnis, Stimme) und/oder seine Aktualisierungstendenz (zum Beispiel die Fähigkeit dieses Bedürfnis aktuell auszuleben/zu äußern.) Die untere Ebene symbolisiert den Bereich der Bedürfnisse (Stimmen...), die nicht aktualisiert und/oder bewußt wahrgenommen werden.
Die mittlere Ebene symbolisiert das innerseelische Geschehen, welches aktualisiert werden kann und in der Regel bewußt ist. Eine dritte obere Ebene könnte zum Beispiel die Stimmen (Bedürfnisse, Stimme, Gedanken) beinhalten, welche nicht primär im Individium entstehen, sondern eher Umweltforderungen darstellen. Der Benutzer kann nun seine innere Konstellation auf den drei Ebenen betrachten und über weitere Einwicklungsmöglichkeiten nachdenken.
Je nach Fragestellung (Problematik, Situation) und psychischer Fähigkeit der Benutzer kann die Anzahl der Ebenen ausgewählt werden.
Bei zwei Ebenen kann auf der mittleren Ebene zum Beispiel auch die empfundenen und internalisierten Umwelterwartungen in den kleinen Stimmenfiguren (21) visualisiert werden.

Weiterhin kann die Spielfigur auch in fester Bauweise konzipiert werden, so daß sie nicht auseinandergebaut und zusammengesteckt werden muß. Hierbei kann, die beschriebene dritte Ebene fest integriert sein.

Die Spielfigur kann weiter so konzipiert sein, daß ein Teil der Holzstange (2) nicht durch den Innenraum der Figur geht und somit weiteren Platz für die Aufstellung der kleinen Stimmenfiguren (21) auf den verschiedenen Ebenen läßt.

Besonders vorteilhaft ist eine Spielfigur aus Holz oder Kunststoff, die aus einem Kopf besteht, in Form einer Holzkugel (1), die im Durchmesser mindestens 70 mm sein sollte, welche auf einer Holzstange (2) in sein Bohrloch (3) aufgestekt wird, und diese Holzstange (2) einen Durchmesser von mindestens 8 mm aufweist und mindestens 200 mm lang ist, und mittig in einen Holzfuß (4) mündet, mit dem sie dauerhaft verbunden ist.

Der Holzfuß sollte einen Durchmesser von mindestens 120 mm und mindestens 20 mm Materialstärke besitzen, um die Standfestigkeit der Visualisierungsfigur zu gewährleisten, und mit einem weiteren aufgeleimten mindestens 20 mm starken und im Durchmesser mindestens 50 mm großen Verstärkungssockel (5) verbunden sein, der die Festigkeit der Holzstange (2) gewährleistet, die mittig einen gedrechselten Hohlkörper (6) und mindestens eine Materialstärke von 5 mm durchläuft.

Der Hohlkörper besitzt die zusammengesetzte Form eines Zylinders und einer Halbkugel, deren Höhe mindestens 180 mm und einen Durchmesser von mindestens 100 mm beträgt, wobei der gedrechselte Hohlkörper (6) einen Raum bildet, der nach hinten und nach vorne geöffnet werden kann.

Weitere Vorteile, Einzelheiten und Merkmale der Erfindung ergeben sich aus der folgenden Beschreibung. Auf der Zeichnung ist die Erfindung beispielsweise abgebildet, und zwar zeigt
- Figur I:: eine perspektivische Darstellung der Visualisierungsfigur mit geschlossenen Türen,
- Figur II:: eine perspektivische Darstellung der Visualisierungsfigur mit geöffneten Türen,
- Figur III:: einen Längsschnitt durch den Hohlkörper (6) und der unteren Ebene (14) zur Darstellung das Falz in der unteren Ebene zur Aufnahme des Hohlkörpers und zum Anschlag der Türen (7),
- Figur IV:: eine Draufsicht auf den Zubehörkasten mit den verschiedenen Visualisierungselementen,
- Figur V:: zeigt eine perspektivische Ansicht einer beispielhaft zusammengesteckten Stimmenfigur (21),
- Abbildung VI:: zeigt eine perspektivische Ansicht verschiedener zusammengesteckter Stimmenfiguren,
- Abbildung VII:: zeigt die Vorlage KU zur weiteren Beschreibung der inneren Konstellation,
- Abbildung VIII:: zeigt das Handbuch zur Visualisierungsfigur,
- Abbildung IX:: zeigt Beispielsschilder zur schriftlichen Fixierung der inneren Stimmen.

Die in Figur I zeigt die perspektivische Ansicht der gesamten Visualisierungsfigur mit geschlossenen Türen. Der Holzkopf (1) der Spielfigur ist mindestens 15 mm tief in einem Bohrloch (3) auf der Holzstange (2) eingesteckt. Auf dem aufgesetzten Holzkopf (1) kann ein Gesichtsausdruck mit einem abwaschbaren Stift aufgetragen werden.
Die Holzstange (2), die in einen Holzsockel (4) mündet, wird zusätzlich durch eine Sockelverstärkung (5) auf dem Holzsockel (4) gesichert und dauerhaft haltbar verbunden. Auf der fest fixierten Ebene (14) liegt der gedrechselte Hohlkörper (6) in einem Falz (15) ein und kann jederzeit von der Holzstange abgenommen werden.
In der Front des zylindrischen Hohlkörpers (6) sind zwei Türen mit innerem Klappscharnier (8) eingearbeitet. Hierbei befinden sich in der Vorderfront auf beiden Türen jeweils ein aufgeleimter Holzknopf (9) zum Öffnen der Türen.
Die festfixierte Fläche (14) hat mindestens einen Abstand von 20 mm zum Verstärkungssockel (5).
Im oberen Bereich (10) der Visualisierungsfigur sehen wir eine Fläche zum Anschlag der Türen und zur Stabilisierung der Spielfigur gemäß der Konstruktionsmerkmale. Die Holzstange (2) durchläuft diesen Bereich in der Mitte. Sein Durchmesser beträgt mindestens 30 mm und hat die Form eines Halbkreises. Diese Halbkreisform wird in den oberen Bereich des Hohlkörpers (6), der an dieser Stelle die Gestalt einer Halbkugel hat, eingearbeitet.

In FIG. II sehen wir die Spielfigur mit geöffneten Türen. In der Mitte des auf gesetzten gedrechselten Hohlkörpers befindet sich eine mittlere Ebene (11), die auf einem kreisförmigen Stutzen (12) aufliegt. Dieser Auflagestutzen (12) stabilisiert und befestigt die mittlere Ebene (11). Hierbei sind die Außenmaße der mittleren Ebene (11) so gewählt, das sie sich in den Innenraum des Hohlkörpers (6) einpaßt. Ihre Fläche ist genauso groß wie die abgefälzte Fläche der unteren festfixierten Ebene (14a) aus dem der Falz (15) zur Aufnahme und Sicherung des Hohlkörpers gearbeitet wird (siehe auch FIG. III).
Der zylindrische Hohlkörper liegt in einem Falz (8) sicher gegen Verrutschen ein. Der Falz (8) ist dabei in die festfixierte Ebene (14) rundum angebracht, damit der aufgesetzte Hohlkörper gesichert ist.
Die Türen (7) mit ihren Türknöpfen (9) werden aus dem Hohlkörper (6) gearbeitet. Sie bilden die gesamte Vorderfront der Spielfigur und bilden die Hälfte des Flächenanteil der Spielfigur. Die Spielfigur mit dem Hohlkörper (6) ist somit zur Hälfte nach hinten geschlossen und in der Vorderfront zur Hälfte durch Türen (7) offen gestaltet.
Die Türen bilden mit dem Hohlkörper (6) eine gemeinsame Fläche. Sie sind mit einfachen Klappschanieren im Inneren des Hohlkörper (6) befestigt und schlagen im geschlossenen Zustand in den Falz (15) und an der verbliebenen Stabilisierungsfläche (10) paßgenau ein und an. Die Beschaffenheit der Türbefestigung ist so gewählt, das die Türen (7) dauerhaft im geschlossenen Zustand verbleiben können.
Auf der mittleren Ebene (11) können im Zuge der Symbolisierung und Visualisierung des innerseelischen Geschehens, die Stimmenfiguren als Symbol für verschiedene Stimmen zu einem klärungsbedürftigen Zustand abgestellt werden. Beispielhaft sehen wir verschiedene Stimmenfiguren auf der mittleren Ebene im Inneren der Spielfigur. Stimmenfigur (21) wird hier exemplarisch bezeichnet. Die Stimmenfigur besteht aus vier Intensitätsbausteinen (23a) mit einem passenden Halterungsstift (19a) auf dem eine Holzkugel (22) aufgesetzt ist. Der Sockel (17) mit dem Bohrloch (18) nimmt den Halterungsstift (19a) auf und stabilisiert die gesamte Stimmenfigur und sorgt für ihre Standfestigkeit. Die übrigen Stimmenfiguren bezeichnen weitere unterschiedliche Stimmen im Inneren der Spielfigur.

In FIG. III sehen wir einen Längsschnitt durch den Hohlkörper (6) und der unteren festfixierten Ebene (14) zur Darstellung des Falz (15), der den Hohlkörper (6) vor dem Verrutschen sichert und den Anschlag für die Türen (7) bildet. Durch den Falz bildet sich die innere Fläche (14a) der unteren Ebene (14) auf der weitere Stimmenfiguren abgestellt werden können.

FIG.IV zeigt die Draufsicht auf den Zubehörkasten (16). Er ist mindestens 45 mm hoch und in ihm können die unterschiedlichen Zubehörelemente zur Visualisierung abgelegt werden. Die jeweiligen Symbole kennzeichnen das Fach für das entsprechende Zubehörelement, die angemessenen Anzahl vorhanden sind. Zur Visualisierung des innerseelischen Geschehens werden verschiedene Elemente in diesem Zubehörkasten (16) zur Verfügung gestellt, welcher so gestaltet ist, das er die angebenden Elemente übersichtlich, praktisch und funktionell in Fächern aufnehmen kann, aus dem der Benutzer die Zubehörteile zu einer kleineren Stimmenfigur (21) je nach seinem Erleben zusammenstecken kann. In ihm sind die unterschiedlichen Formen der Intensitätsbausteine, in runder (23a), in quadratischer (23b), in achteckiger (23c) und in dreieckiger Form (23d) vorhanden. Weiterhin befinden sich Sockelfüße (17) die Halterungsstifte (22a,b,c,d,e,f) in großer Anzahl in dem Zubehörkasten. Die Holzkugel (22a,b,c,d,e,d) mit den aufgetragenen Gesichtern sind in sechs Ausführungen vorhanden. Der Benutzer kann auch die mit einem Faserstift eine unbezeichnete Holzkugel (22) aus dem Zubehörkasten entnehmen. Weiterhin finden wir hier ein Fach, welches die Papierschilder (24) aufnimmt, damit der Benutzer seiner symbolisch visualisierten Figur eine Beschreibung geben kann.

In FIG.V sehen wir ein Anwendungsbeispiel dafür, wie eine Stimmenfigur zusammengesetzt wird. In den Holzsockel (17) wird der Halterungsstift (19) in ein Bohrloch (18) eingedrückt. Je nach Länge des Halterungsstiftes werden verschiedene Intensitätsbausteine, z. B. (23a) in einer gewissen Anzahl auf den Halterungsstift aufgeschoben. Der überstehende Teil des Halterungsstiftes nimmt den kleineren Holzkopf (22) der Stimmfigur auf.

Abb. VI zeigt exemplarisch verschiedene Stimmenfiguren, die aufgrund des Erlebens des Benutzers gesteckt werden können. Wir erkennen den Sockel (17), der einen Halterungsstift (19) aufnimmt und von dem vier verschiedene Längen vorhanden sind (19a,b,c,d). Weiterhin sehen wir die verschiedenen Intensitätsbausteine in runder (23a), quadratischer (23b), in achteckiger (23c) und in dreieckiger Form (23d).

Die erste abgebildete Stimmenfigur A trägt 4 runde Intensitätsbausteine (23a) mit dem größten Halterungsstift (19a) auf dem der Gesichtsausdruck (22c) aufgesetzt worden ist.
Die Stimmenfigur B besitzt z. B. nur einen quadratischen Intensitätsbaustein (23b) mit entsprechenden Halterungsstift (19d). Dieser Stimmenfigur wurde exemplarisch eine unbezeichnete Gesichtsholzkugel (22) aufgesetzt, die der Benutzer selbst aufzeichnen kann.
Die Stimmenfigur C trägt zwei achteckige Intensitätsbausteine (23c) mit entsprechenden Halterungsstift (19c). Dieser Stimmenfigur wurde der Gesichtsausdruck (22d) aufgesetzt.
Stimmenfigur D trägt drei dreieckige Intensitätsbausteine (23d) mit Halterungsstift (19b) dem der Gesichtsausdruck (22a) aufgesetzt ist.
Dies ist nur ein kleiner Ausschnitt aus den Gestaltungsmöglichkeiten zur Symbolisierung der möglichen erlebten Stimmen im innerseelischen Geschehen des Benutzers.

Abb. 7 zeigt die Vorlage KU. Sie hat die Größe einer DIN A4 Seite. Sie kann der Benutzer als Hilfestellung gebrauchen. Handelt es sich um eine konkret erlebte Situation kann der Benutzer nach der ersten Selbstwahrnehmungsübung weiter darüber nachdenken welche Stimmen den Kontakt in der Situation gestaltet haben und welche Stimmen eher im Hintergrund geblieben sind, um Veränderungsmöglichkeiten zu entwickeln.

Abb. 8 zeigt die Draufsicht auf das Handbuch zur Visualisierungsfigur.
Dieses Handbuch beschreibt in kurzer, knapper Form die Grundlagen der Visualisierungsfigur. Wesentliche Aspekte der Persönlichkeitserklärung werden hier erläutert.
Weiterhin werden wesentliche Aspekte Entwicklungspsychologie dargestellt.

In Abb. 9 sehen wir exemplarisch beschriftete Papierschilder, die der Benutzer als Hilfestellung zur schriftlichen Fixierung seiner Stimmen benutzen kann. Symbolisch visualisiert werden kann nur immer das innerseelische Geschehen des Benutzers mit seiner inneren Stimmenaufstellung zu einer konkreten Situation in der er sich verhalten hat oder sich verhalten wird. Oder der Benutzer der Spielfigur möchte seinen augenblicklichen Stimmungszustand reflektieren. Zu jeder Situation, die wir erleben bilden und formieren sich verschiedene Stimmen in unserer seelischen Geschehen.

Der Benutzer stellt sich die Spielfigur mit dem Zubehörkasten vor sich auf einen Tisch.
Der Benutzer sieht vor sich die Visualisierungsfigur.
Er schöpft für einen Augenblick Ruhe und versucht sich zu sammeln und zu entspannen.
Er fühlt nun die Figur. Die Figur, die er fühlt ist er selbst. Er möchte nun die verschiedenen Stimmen erfahren, die seine augenblickliche seelische Verfassung ausmachen oder was eine konkret erlebte Situation für unterschiedliche Stimmen in seinem innerseelischen Geschehen ausgelöst haben.
Er beschreibt wie sie dazu gekommen ist eine Klärung über seinen inneren Zustand vorzunehmen. Der Benutzer beschreibt ruhig die Vorgeschichte oder Situation, wie es zu diesem klärungsbedürftigen Zustand gekommen ist, der ihn im Augenblick beschäftigt. Mögliche Fragen: Welche Entscheidung möchte ich treffen? Was möchte ich verändern? Welche Empfindungen habe ich in dieser konkreten Situation?
Der Benutzer nimmt nun den großen Holzkopf (1) in die Hand und versucht einen passenden Gesichtsausdruck für seine augenblickliche Befindlichkeit mit einem abwaschbaren Faserstift aufzutragen. Ist dies im Augenblick nicht möglich, kann dies auch später gemacht werden.
Er versucht nun eine konkrete Frage zu formulieren, worüber er Klarheit bekommen möchte. Er trägt diese Frage auf eine das Papierschild (24) ein und beginnt dann die Beschreibung im lauten Selbstgespräch seiner inneren Stimmen. Er formuliert:

"In mir ist als erstes eine Stimme/ Person/, die sagt, ... die möchte... die wünscht sich... die fühlt sich...

Nach einer kurzen Beschreibung wählt er die entsprechende Form (23) aus dem Zubehörkasten, von denen er meint, daß es auf ihre erste Stimme passen würde. Er beschreibt weiter sehr genau die erste Stimme. Nach dem er den entsprechenden Intensitätsbaustein (23) ausgewählt hat, versucht er im lauten Selbstgespräch zu beschreiben, die Form des Oberkörpers (23) gewählt haben. Er versucht weiterhin die Intensität dieser Stimme zu bestimmen. Er wählt die Anzahl der Intensitätsbausteine (23) seiner Stimmenfigur und beschreibt weiter in welcher Intensität diese Stimme/dieses Bedürfnis/diese Stimme vorhanden ist. Hierfür nimmt er eine entsprechende Anzahl von Bausteinen, die seiner empfundenen Intensität entspricht. Er kann zwischen 1 bis 4 Intensitäten wählen. Der Benutzer denkt darüber nach warum er gerade diese Form (23) für diese Stimme gewählt hat. Wichtig hierbei ist nur seine subjektive Bedeutung der Stimmen. Er kann auch unterschiedliche Intensitätsbausteine (23a,b,c,d) zu einer Stimmenfigur zusammenstecken. Weiterhin entnimmt er aus dem Zubehörkasten die entsprechende Länge des Halterungsstiftes (19). Der Benutzer steckt praktisch unter seinem lauten Selbstgespräch seine erste Stimmenfigur zusammen. Wenn er das Gefühl hat, seine Stimme genügend umschrieben zu haben, versucht er dieser Stimme eine kurze Beschreibung zu geben. In Abb. 9 sehen wir eine Möglichkeit solch eine Umschreibung zu treffen. Er nimmt aus dem Zubehörkasten (16) ein Papierschild (24) fixiert schriftlich, das Hauptanliegen, das Motiv, das Bedürfnis, das Erleben, das Gefühl dieser Stimme. Der Benutzer nimmt nun seine erste zusammengesetzte Stimmenfigur (21) und stellt sie in den inneren Raum der Visualisierungsfigur. Er öffnet die beiden Türen (7) und stellt die erste Figur auf die mittlere Ebene (11). (siehe FIG.II)
Nachdem er die erste Stimmenfigur in die Figur gestellt hat, schließt er wieder beide Türen. Er sagt noch einmal welche Stimme er dort symbolisch für sein inneres Geschehen abgestellt hat und legt das Papierschild (24) mit seiner Beschreibung vor die Visualisierungsfigur.

Im folgenden beginnt er weiter seine zweite Stimme zu beschreiben. Der Benutzer vergegenwärtigt sich immer wieder seine Fragestellung und durchläuft den beschriebenen Vorgang solange bis er alle Stimmen den ihnen einfallen beschrieben hat. Es ist jeder Zeit erlaubt innere Stimmen zu verändern oder zu entfernen solange dies im lauten Selbstgespräch passiert.
Wenn der Benutzer jedesmal seine Stimme ausführlich beschrieben hat, stellt er die zusammengesteckte Figur von Gesicht und Intensitätsbausteinen in die Visualisierungsfigur und schließt jedes Mal die Türen der Figur als symbolischen Akt, das dies eine innere Stimme in seinem inneren Geschehen zu der Fragestellung ist.
Sind alle Stimmen in dem Klärungszusammenhang beschrieben befinden sich eine gewisse Anzahl von Stimmenfiguren (21) im Inneren der Visualisierungsfigur und die verschiedenen Papierschilder vor der Spielfigur.
Nun kann der Benutzer seine innere Stimmenvielfalt betrachten. Er nimmt den Holzkopf (1) von der Spielfigur und zieht den Hohlkörper (6) von der der Spielfigur. Er kann nun seine Stimmenvielfalt zu dem klärungsbedürftigen Zustand betrachten.
Handelt es sich um eine Selbstwahrnehmung bezüglich der augenblicklichen Stimmung kann der Benutzer die Stimmen noch einmal versuchen zu beschreiben durch welche inneren und äußeren Bedingungen die Stimmen ausgelöst worden sind. Hierbei stellt er den Hohlkörper (6) wieder auf die festfixierte Ebene (11). Die Stimmenfiguren werden nun noch mal einzeln aus dem Inneren genommen.
Der Benutzer fragt sich aus welchen Gründen sich diese Stimme so fühlt, so denkt, so motiviert ist.

Wenn der Benutzer eine konkrete Situation in der er sich verhalten in seinem inneren Erleben exploriert hat, kann er weiter mit Vorlage KU mit der Außenwelt gestaltet haben. Und welche Stimmen standen eher im Hintergrund und wären vielleicht doch gerne zur Sprache gekommen und wären verwirklicht worden. Und aus welchen Gründen wurde gerade diese Stimmen nach vorne geschickt und warum wurde diese Stimme in den Hintergrund gedrängt, obwohl man sich in der Situation lieber anders verhalten hätte.
Und dies ist jetzt ja möglicherweise der Grund, warum man sich unwohl fühlt. In diesem Zusammenhang kann der Benutzer auch die Vorlage KU benutzen, um die einzelnen Stimmen seiner Stimmung aufzustellen. Wiederholt er seine Selbstwahrnehmungsprozeße, kann er durch Vorlagenvergleich feststellen, ob es in der Aufstellung des inneren Geschehens eine Veränderung gegeben hat (Abb./).

Die Spielfigur kann z. B. auch von zwei Menschen anläßlich einer konflikthaften Situation genutzt werden. Durch die parallele Darstellung der individuellen inneren Konstellation können die beiden Konfliktpartner in ein konfliktbearbeitendes Gespräch kommen.
Hierbei beschreibt z.B. der Partner sein innerseelisches Geschehen zu einer konkreten Situation oder eines
Problems, welches schriftlich in Übereinstimmung von beiden Partnern auf dem Papierschild festgelegt worden ist.
Der erste Gesprächspartner beginnt in oben genannter Ausführungsform seine innere Stimme zu beschreiben und stellt diese vor die Spielfigur. Während er erzählt, darf der andere ihn nicht unterbrechen. Der Gesprächspartner, welcher seine Figur zusammensteckt, äußert sich solange zu seiner ersten inneren Stimme bis er das Gefühl hat, diese ausführlich erklärt zu haben.
Der Zuhörende soll nur erst einmal zuhören und versuchen, zu verstehen, welche Merkmale, Gefühle, Gedanken diese erste Stimme beinhaltet.
Die vor der Spielfigur abgestellte Stimmenfigur z. B. (21) wird nun vom anderen zuhörenden Gesprächspartner beschrieben. Der zuhörende Partner hat nun die Aufgabe die erste Stimmenfigur seines Gesprächspartners zu beschreiben. Er versucht Verständnis für die erste Stimme seines Gesprächspartners zu entwickeln und diese zu begreifen. Er beschreibt die andere Stimme mit seinen Worten. Erst wenn der andere Gesprächspartner, welcher seine erste Stimme beschrieben hat, sich vom anderen in dieser Stimme verstanden fühlt, wird sie in das Innere der Spielfigur abgestellt.
Nun wechseln die Rollen. Der zuhörende Partner hat nun die Möglichkeit seine erste Stimme zu dem gemeinsam definierten Problem zu beschreiben. Auch er hat ersteinmal das unbedingte Recht seine erste Stimme zum Problem zu beschreiben, ohne unterbrochen zu werden. Es ist sehr wichtig diese Regel einzuhalten. Der beschreibende Gesprächspartner hat das Recht, das ihm unbedingt zugehört wird.
Der andere zuhörende Gesprächspartner muß dann versuchen diese Stimmenfigur nochmals zu beschreiben und stellt diese dann in die Spielfigur des Partners ab, wenn der Partner sich in seiner ersten Stimme verstanden fühlt.

An den Stellen, wo der sich erklärende Partner sich nicht durch die Ausführungen des zuhörenden und dann beschreibenden Gesprächspartners verstanden fühlt, hilft er den zuhörenden Partner um diese Stimme zu verstehen.
Jegliche Beschuldigungen, Schuldzuweisungen, Deutungen, Interpretationen zerstören in dieser Phase den kommunikativen Austausch. Alte Teufelskreise der Kommunikation und des Erlebens wieder durch diese Verhaltensweisen wieder aktiviert.
Durch diese spielerische Auseinandersetzung können so je nach Konfliktfähigkeit, gegenseitiger Akzeptanz und Werthaltung der beteiligten Personen problematische Wechselwirkungen im Beziehungs- und Kommunikationsgeschehen erkannt offengelegt werden.
Auch wäre es möglich die Spielfigur zum Gespräch mit einer vertrauten Person zu benutzen. Hierbei beschreibt der Benutzer nicht sich selbst im lauten Selbstgespräch seine inneren Stimmen, sondern hat einen Gesprächspartner, der ihm zuhört und Fragen zum Verständnis seiner inneren Stimmen stellen kann.
Allerdings sollte der Benutzer überlegen, in welchem Verhältnis er zu seinem zuhörenden Gesprächspartner steht und welche Fragestellung er mit welcher Person bei seiner Selbstwahrnehmungsübung angehen möchte. Diese muß er dann im individuellen Fall für sich entscheiden.
Weiterhin kann die Spielfigur im Rahmen eines therapeutischen Settings sinnvoll eingesetzt werden.
Durch die fachliche Intervention wird die Selbstwahrnehmung intensiver gestaltet. Besonders bei Beziehungsklärungen kann hier unter fachlicher Intervention der Klärungsprozeß gestaltet werden.

Wenn es sich um die Frage einer möglichen Verhaltensänderung handelt, überlegen der Benutzer oder die Benutzer gemeinsam welche Stimmen sie entwickeln und welche Stimmen in ihrer Intensität verringern möchten, um mit sich und im Kontakt mit anderen sich wohler zu fühlen, also ihren spannungsreichen Zustand zu reduzieren.

Zur Initiative einer Verhaltensänderung müssen verschiedene Aspekte betrachtet werden: Grundsätzlich ist der Anspruch dieser Visualisierungsfigur dort zu sehen, wo der Prozeß der Selbstwahrnehmung förderlicher gestaltet werden kann. Dieser Prozeß bildet erst die Grundlage dafür, wahrzunehmen, was in ihm passiert, welche Gedanken, Gefühle und Motivationen anläßlich einer konkreten Situation in ihm vorhanden sind.

Dies ist erst einmal die Grundlage dafür, daß er mit seiner Umwelt angemessen in Kontakt treten kann. Eine unklare, nicht bewußte oder nur teilweise bewußte oder starre innere Konstellation kann zu Störungen in der Kommunikation mit anderen Menschen führen. Hat der Benutzer das, was sich wichtig ist geklärt, kann es dies auch adäquater seine Umwelt mitteilen.
In diesem Moment tritt der Benutzer oder die Benutzer praktisch in den Kommunikationsprozeß ein.
Weiterhin ist zu klären, welche Kommunikationsregeln in der Beziehung, in der ein Problem besteht, herrschen und welche systematischen Strukturen und Gesetzmäßigkeiten der Interaktion sich entwickelt haben.
Wenn wir auf diesem Hintergrund versuchen, eine Veränderung herbeizuführen, können wir das erst einmal nur innerhalb unseres ganz individuellen Rahmens. Bei mehr als einem Benutzer wird die Klärung des gemeinsam entwickelten Rahmen unterstützt.

## Patentansprüche

1. System mit mindestens einer Spielfigur (1) als Mittel zur Symbolisierung und Visualisierung des innerseelischen Geschehens in Einzelpersonen zur Übung der Selbstwahrnehmung und Bewußtmachung von Konflikten in Zweierbeziehungen und
Gruppenbeziehungen, wobei das System mindestens einen Symbolträger (21) und die Spielfigur (1) einen Hohlraum zur Aufnahme von Symbolträgern (21) aufweist, der vorzugsweise durch eine Tür 87) zugänglich ist, wobei die Tür (7) an der Vorderseite der Spielfigur (1) eingearbeitet ist und wobei die Spielfigur (1) das Äußere eines Menschen aufweist, **dadurch gekennzeichnet, daß** der Hohlraum mehrere Ebenen zur Aufstellung von Symbolträgern (21), insbesondere von Stimmenfiguren (21), aufweist.

2. System mit mindestens einer Spielfigur nach Anspruch 1, **dadurch gekennzeichnet, daß** die Symbolträger (21) aus Einzelteilen zusammensetzbar ausgebildet sind.

3. System mit mindestens einer Spielfigur nach Anspruch 1 oder 2, **dadurch gekennzeichnet, daß** mindestens ein Symbolträger (21), insbesondere eine kleinere Stimmenfigur (21) je nach seinem Erleben gestaltbar, zum Beispiel in der Größe variierbar, ausgebildet ist.

4. System mit mindestens einer Spielfigur nach Anspruch 1, 2 oder 3, **dadurch gekennzeichnet, daß** die Stimmenfiguren mittels Halterungsstiften (12) in, vorzugsweise vier, verschiedenen Längen (19 a, b, c, d) in den Sockel (17) der Stimmenfigur (21) eingesteckt werden können und ihre Größe je nach Anzahl aufgesteckter Intensitätssteine (23a, b, c, d) variierbar sind und/oder zu den gewählten Intensitätssteinen (23a, b, c, d) Kopfteile (22) mit einem bestimmten Gesichtsausdruck auf die Intensitätsbausteine aufsetzbar sind, insbesondere verschiedene, vorzugsweise sechs, Gesichtsausdrücke vorgesehen sind und/oder mit einem Schreibinstrument, vorzugsweise abwaschbaren Faserstift, selbst auf einer vorgesehenen Fläche darstellbar sind.

5. System mit mindestens einer Spielfigur nach Anspruch 1, 2, 3 oder 4, **dadurch gekennzeichnet, daß** Kopfteile (22), vorzugsweise Holzkugeln (22), ohne Gesichtsausdruck vorgesehen sind.

6. System mit mindestens einer Spielfigur nach Anspruch 1, 2, 3, 4 oder 5, **dadurch gekennzeichnet, daß** als Symbolträger Schilder (24) vorzugsweise aus Papier, zur schriftlichen Fixierung von Stimmen vorgesehen sind.

## Claims

1. A system with at least one play figure (1) as a means for symbolising and visualising the psychological events in individuals for exercising introspection and raising the awareness of conflicts in pair relationships and group relationships, wherein the system comprises at least one symbol carrier (21) and the play figure (1) comprises a cavity for accommodating symbol carriers (21), which is preferably accessible through a door (7), wherein the door (7) is incorporated on the front side of the play figure (1) and wherein the play figure (1) has the outer appearance of a human, **characterised in that** the cavity comprises several levels for erecting symbol carriers (21), in particular sentiment figures (21).

2. A system with at least one play figure according to claim 1, **characterised in that** the symbol carriers (21) are designed able to be composed of individual parts.

3. A system with at least one play figure according to claim 1 or claim 2, **characterised in that** at least one symbol carrier (21), in particular a smaller sentiment figure (21) is designed such that it may be fashioned according to its experiences, for example may be varied in size.

4. A system with at least one play figure according to claim 1, 2 or 3, **characterised in that** the sentiment figures, by way of retaining pins (12) in, preferably four, various lengths (19 a, b, c, d), may be inserted into the base (17) of the sentiment figure (21), and their size may be varied according to the number of intensity pieces which are placed on (23a, b, c, d), and/or for the selected intensity pieces (23a, b, c, d), head parts (22) with a certain facial expression may be placed on the intensity pieces, in particular various, preferably six, facial expressions are provided, and/or with a drawing instrument, preferably an erasable felt pen, may themselves be represented on a provided surface.

5. , A system with at least one play figure according to claim 1, 2, 3, or 4, **characterised in that** head parts (22), preferably wooden balls (22) are provided without a facial expression.

6. A system with at least one play figure according to claim 1, 2, 3, 4 or 5, **characterised in that** signs (24) preferably of paper are provided for the written fixing of sentiments

## Revendications

1. Système avec au moins une figure de jeu (1) comme moyen pour symboliser et visualiser l'état psychique intérieur de personnes individuelles, en vue d'exercer l'auto-perception et la conscienciation de conflits dans les relations à deux et les relations de groupe, le système présentant au moins un porte-symbole (21) et la figure de jeu (1) une cavité destinée à recevoir des porte-symbole (21) accessible, de préférence, à travers une porte (87), la porte (7) étant pratiquée du côté avant de la figure de jeu (1) et la figure de jeu (1) présentant l'extérieur d'un être humain, **caractérisé par le fait que** la cavité présente plusieurs niveaux pour l'installation de porte-symbole (21), en particulier de figures d'humeur (21).

2. Système avec au moins une figure de jeu selon la revendication 1, **caractérisé par le fait que** les porte-symbole (21) sont réalisés de manière à pouvoir être assemblés à partir de pièces individuelles.

3. Système avec au moins une figure de jeu selon la revendication 1 ou 2, **caractérisé par le fait qu'**au moins un porte-symbole (21), en particulier une petite figure d'humeur (21), peut être formé selon son expérience, par exemple de manière variable en grandeur.

4. Système avec au moins une figure de jeu selon la revendication 1, 2 ou 3, **caractérisé par le fait que** les figures d'humeur peuvent être introduites, à l'aide de goupilles de support (12), selon, de préférence quatre, longueurs différentes (19a, b, c, d) dans le socle (17) de la figure d'humeur (21) et que leur grandeur est variable selon le nombre de modules d'intensité (23a, b, c, d) et/ou que sur les modules d'intensité (23a, b, c, d) choisis peuvent être enfilés des éléments de tête (22) à expression de visage déterminée, en particulier qu'il est prévu des expressions de visage différentes, de préférence six, et/ou qu'elles peuvent être représentées à l'aide d'un instrument d'écriture, de préférence un feutre effaçable, sur une face prévue à cet effet.

5. Système avec au moins une figure de jeu selon la revendication 1, 2, 3 ou 4, **caractérisé par le fait qu'**il est prévu des éléments de tête (22), de préférence des billes en bois (22), sans expression de visage.

6. Système avec au moins une figure de jeu selon la revendication 1, 2, 3, 4 ou 5, **caractérisé par le fait qu'**il est prévu, comme porte-symbole, des étiquettes (24), de préférence en papier, pour la fixation par écrit d'humeurs.
